# EUROPÄISCHE PATENTSCHRIFT

(11) **EP 0 451 228 B1**
(45) Veröffentlichungstag und Bekanntmachung des Hinweises auf die Patenterteilung: **28.12.1994**
(21) Anmeldenummer: 90914065.9
(22) Anmeldetag: 28.09.1990
(51) Int. Cl.: C08K 5/34, C08K 5/42, C08K 5/00, C07D 401/12

(54) **AUFHELLER- UND LICHTSTABILISATOR-SALZE**
BRIGHTENING AND LIGHT-STABILIZING SALTS
SELS D'AZURAGE OPTIQUE ET DE PHOTOSTABILISATION

(30) Priorität: 03.10.1989 DE 3932914
(43) Veröffentlichungstag der Anmeldung: 16.10.1991
(73) Patentinhaber: SANDOZ-PATENT-GMBH, 79539 Lörrach (DE); SANDOZ LTD., 4002 Basel (CH)
(72) Erfinder: KAUL, Bansi, Lal, CH-4105 Biel-Benken (CH); VOUGIOUKAS, Angelos-Elie, F-68300 S.-Louis (FR)
(86) Internationale Anmeldenummer: CH9000230
(87) Internationale Veröffentlichungsnummer: WO9104987

(56) Entgegenhaltungen:
- EP-A- 0 094 642
- EP-A- 0 117 229
- EP-A- 0 310 083
- JP-A- 7 558 141
- Journal of Applied Polymer Science, Band 32, Nr. 4, September 1986 John Wiley & Sons, Inc.,G.N. Sheth:"Studies in interaction between polyvinyl pyrrolidone and stilbene fluorescent compounds II. Interaction with mixture of compounds", pages 4333-4349, see page 4334, coumpound 32

## Beschreibung

Es hat sich gezeigt, dass in der Masse Aufheller, bzw. Lichtstabilisatoren enthaltendes Fasermaterial aus synthetischen Polyamiden schlechte Nassechtheiten, insbesondere eine schlechte Waschechtheit, besitzt, die Aufheller und Lichtstabilisatoren diffundieren in die Waschflotte unverhältnismässig stark aus, was zu Protesten der Verbraucher geführt hat.

Im Zuge der Arbeiten zur Beseitigung dieses Mangels wurde gefunden, dass die Aufheller und Lichtstabilisatoren der allgemeinen Formel I

Aₙ .(B)ₙ (I),

worin
- A: den Rest eines a) anionischen oder b) kationischen Aufhellers oder Lichtstabilisators,
- B: im Fall a) einen mindestens eine Ammonium-, Cyclosmmonium- oder Immonium-Gruppe und mindestens eine aliphatische, cycloaliphatische, an mindestens eine (bis zu vier) Carbonylgruppe gebundene aromatische oder eine heteroaromatische Gruppe aus der Reihe der Triazin-, Pyrimidin-, Chinazolin-, Chinoxalin-, Piperazin-, Phthalimid oder Phthalazinverbindungen enthaltender Rest, wobei die eine aliphatische oder cycloaliphatische Gruppe enthaltenden Reste auch mindestens eine cyclisch gebundene Ammoniumgruppe enthalten, und im Fall b) einen mindestens einen Carbonsäure- oder Sulfonsäurerest und eine aliphatische, cycloaliphatische, aromatische oder heteroaromatische Gruppe aufweisenden Rest oder einen C₂₋₁₄-Dicarbonsäure- oder Amino-C₂₋₁₂-alkylcarbonsäurerest und die beiden n unabhängig voneinander 1, 2, 3 oder 4 bedeuten, wobei A und B salzartig aneinander gebunden sind, sich zum Aufhellen, bzw. Stabilisieren von Kunststoffen, vor allem synthetischen Polyamiden, insbesondere zu Fasermaterial zu verarbeitenden synthetischen Polyamiden, ausgezeichnet eignen. Die Erfindung betrifft also diese Verbindungen, ihre Herstellung und die genannte Verwendung.

Die Aufhellungs- bzw. Stabilisierungseffekte mit diesen Verbindungen sind sehr markant, sie sind ganz wesentlich nassechter, insbesondere waschechter als mit den entsprechenden, nicht salzartig an Reste B gebundenen Verbindungen. Die erfindungsgemässen Verbindungen eignen sich auch hervorragend für das

Aufhellen oder Lichtstabilisieren löslicher Kunststoffe, z.B. Nitrocellulose-Lacken.

Die Aufheller- und Lichtstabilisator-Reste A können anionischen oder auch kationischen Charakter besitzen. Reste A von anionischen Aufhellern sind im allgemeinen bevorzugt. Gleichermassen bevorzugt sind auch die Reste B, die bis zu vier, vorzugsweise eine oder zwei Ammonium- oder Immoniumgruppen, (worunter auch cyclisch gebundenes Ammonium, insbesondere in 2,2,6,6-Tetramethylpiperidin- oder in Piperazinresten gebundener Ammonium-Stickstoff zu verstehen ist) und ein C₁₋₁₂-Alkyl-, Cyclohexan-, Piperazin-, Triazin-, Pyrimidin-, Chinazolin-, Chinoxalin-, Phthalazin-, Phthalimid- oder Benzol-Brückenglied, vorzugsweise ein Brückenglied der Benzol- oder Triazinreihe enthalten. Bevorzugt sind damit auch die Verbindungen der Formel I, worin ein n 1 und das andere n 1 oder 2, insbesondere 1 ist.

Unter den Resten von Aufhellern A sind vor allem diejenigen der Formel 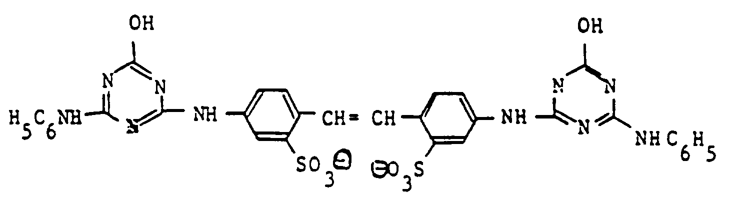
sowie die im Buch von K. Venkataraman "The Chemistry of Synthetic Dyes" Vol.V, 1971 auf den Seiten 550-674 beschriebenen, saure Gruppen (-SO₃H-Gruppen) oder basische Gruppen (z.B. Ammonium-, Oxazolium-, Thiazolium-, Pyridinium- oder Piperidiniumgruppen) enthaltenenden Verbindungen bevorzugt.
Unter den Lichtstabilisatoren, vor allem UV-Stabilisatoren, sind insbesondere die aus Kirk-Othmer "Encyclopedia of Chemical Technology" 3rd Edition, Vol. 23, pp. 615-625 bzw. die aus "Ullmanns Encyklopädie der technischen Chemie", 4. Auflage, Bd. 15, Seiten 253-267, i.b. 257-260, bekannten Verbindungen, insbesondere in sulfonierter Form, zu verstehen.

Bevorzugt als Verbindungen B (freie Amine, bzw. Imine, noch nicht salzförmig gebunden) sind solche, die eine oder mehrere gleichzeitig als Lichtstabilisator wirkende Reste mit sterisch gehindertem Aminostickstoff enthalten, insbesondere Gruppen der Formeln a bis e enthalten 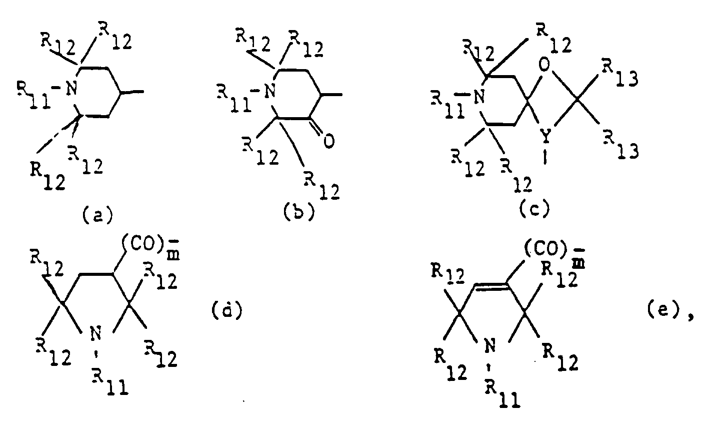
worin
- R₁₁: Wasserstoff oder C₁₋₄-Alkyl, vorzugsweise Wasserstoff oder C₁₋₂-Alkyl,
alle
- R₁₂: unabhängig voneinander C₁₋₅-Alkyl, vorzugsweise Methyl,
- Y-: eine Gruppe der Formel -CO-N< oder >N-CO-
die beiden R₁₃ unabhängig voneinander Wasserstoff, C₁₋₂-Alkyl oder ein
- R₁₃: Phenyl, das andere Wasserstoff oder C₁₋₂-Alkyl oder beide
- R₁₃: zusammen eine Gruppe der Formel -(CH₂))₁₁-
und
- m: 0 oder 1 bedeuten,
wobei die Verbindungen mit einer Gruppe der Formel a bevorzugt sind. Bevorzugt sind damit die Verbindungen der Formel I, worin A der Rest eines anionischen Aufhellers und B ein Rest ist, der eine oder zwei 2,2,6,6-Tetramethylpiperidinium oder 1,2,2,6,6-Pentamethylpiperidiniumgruppen und gegebenenfalls eine, zwei oder drei 2,2,6,6-Tetramethylpiperidylgruppen oder 1,2,2,6,6-Pentamethylpiperidylgruppen, beide in 4-Stellung gebunden, trägt.

Besonders bevorzugt sind auch die Verbindungen der Formel I, worin B mit einer, zwei oder drei Gruppen der Formel f und einer über eine -CO-Brücke gebundenen Gruppe der Formel g 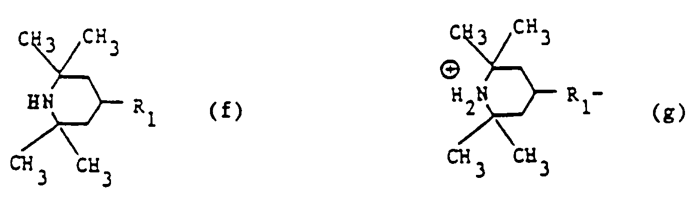
(R₁ = -O- oder -NR₂- und R₂ = H oder C₁₋₂-Alkyl)
substituiertes Benzol, oder Triazin, das in 2-Stellung eine Gruppe der Formel g, in 4-Stellung eine Gruppe der Formel f und in 6-Stellung ein Chlor oder ebenfalls eine Gruppe der Formel f als Substituenten trägt, oder eine Verbindung der Formel 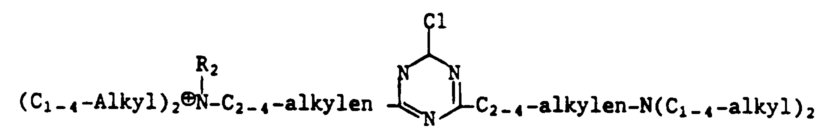
worin R₂ Wasserstoff oder C₁₋₃-Alkyl, vorzugsweise Wasserstoff und n 1 bedeuten.

Weitere Beispiele für bevorzugte Komponenten B (als freie Amino- bzw. -cyclische Aminoverbindungen) sind:
1. 1,3,5-Tri-(2',2',6',6'-tetramethylpiperidyl-4')-trimesinsäuretriamid,
2. 2,4-Bis-(2',2',6',6'-tetramethylpiperidyl-4'amino)-6-chlortriazin,
3. 2,4,6-Tri-(2',2',6',6'-tetramethylpiperidyl-4'amino)-triazin,
4. Bis-(2 ,2 ,6 ,6 -tetramethylpiperidyl-4'aminocarbonyl -paraphenylen)-terephthalsäurediamid,
5. Bis-(2 ,2 ,6 ,6 -tetramethylpiperidyl-4')-terephthalsäurediamid,
6. 2,4-Bis-(2',2',6',6'-tetramethylpiperidyl-4'amino)-chinazolin,
7. 2,3-Bis-(2',2',6',6',-tetramethylpiperidyl-4'amino)-chinoxalin,
8. 1,4-Bis-(2',2',6',6',-tetramethylpiperidyl-4'amino)-phthalazin,
9. 2-Chlor-4,6-Bis-(2',2',6',6',-tetramethylpiperidyl-4'-amino)-pyrimidin,
10. 2,5-Dichlor-4,6-Bis-(2',2',6',6',-tetraethylpiperidyl-4'-amino)-pyrimidin,
11. 2-Fluor-5-chlor-4,6-Bis-(2',2',6',6',-tetraethylpiperidyl-4'-amino)-pyrimidin,
12. 2,4,6,Tri-(2',2',6',6',-tetriethylpiperidyl-4'-amino)-pyrimidin,
13. 2,4,6,Tri-(2',2',6',6',-tetramethylpiperidyl-4'-amino)-5-chlor-pyrimidin, sowie die Verbindungen der Formeln
14. 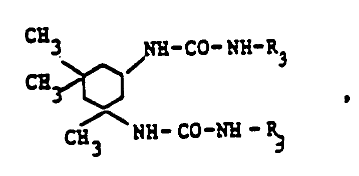
15. 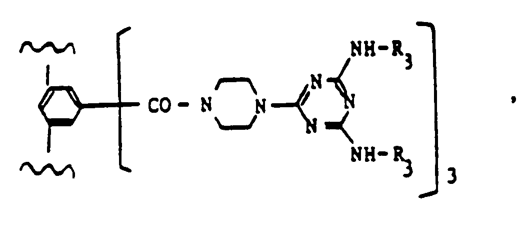
16. 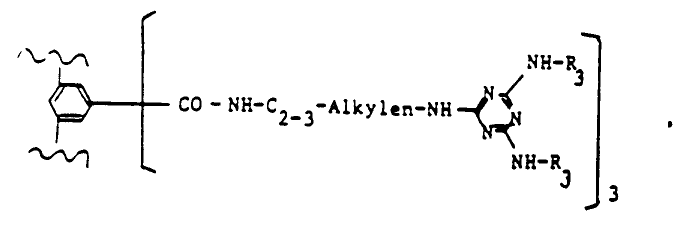
17. 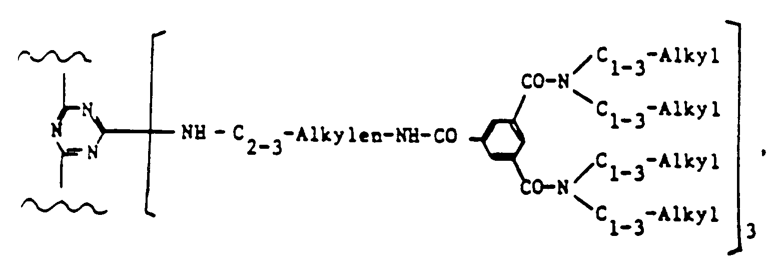
18. 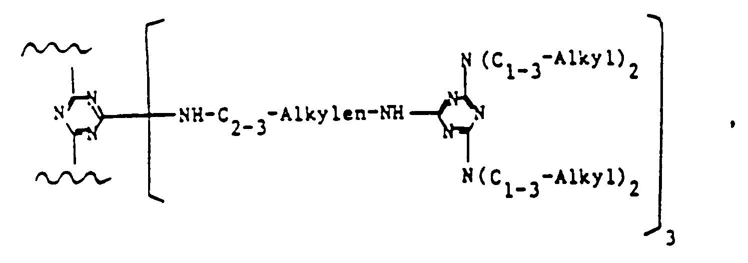
19. 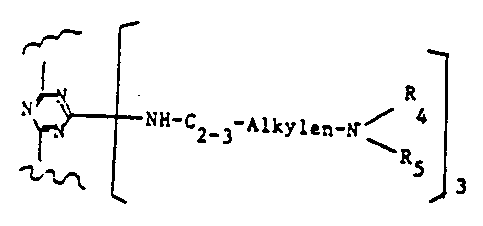
20. 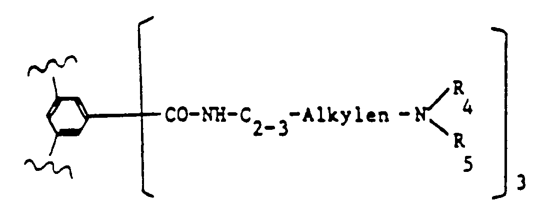
   - R₄ und R₅: bedeuten in Formel 19 und 20 unabhängig voneinander Wasserstoff oder C₁₋₃ -Alkyl,
21. 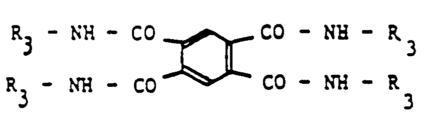
22. 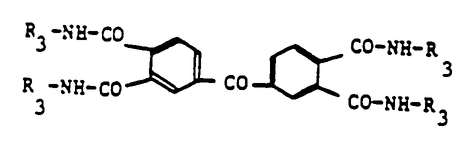
23. 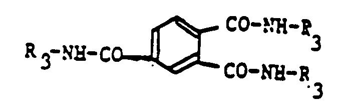
24. 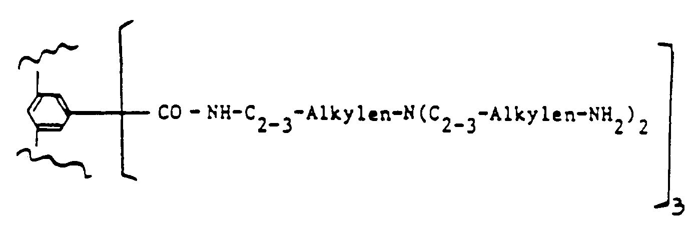
25. 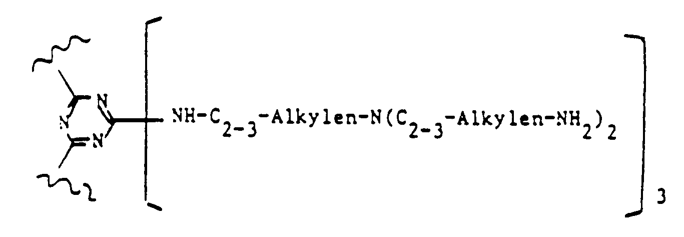
26. 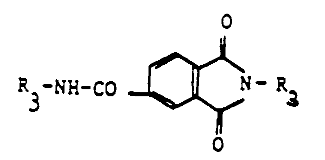
27. 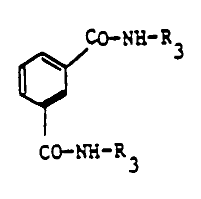
28. 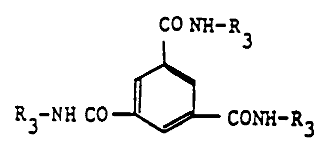
29. 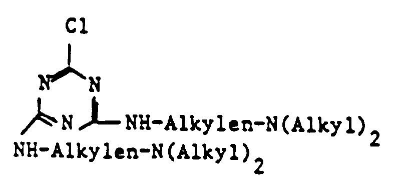
30. 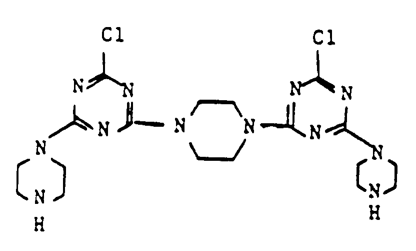
31. 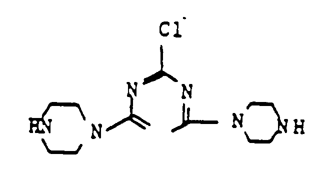
32. 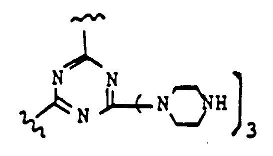
33. R₆(-CO NH - R₃)ₚ
34. H₂N-C₂₋₁₂-Alkylen CONH-R₃, worin p 1, 2, 3 oder 4, vorzugsweise 1 oder 2, R₃ 2,2,6,6-Tetramethylpiperidyl-4- und R₆ einen p-wertigen aliphatischen Rest mit bis zu 12, vorzugsweise einen Alkylrest mit bis zu 6, insbesondere 1, 2, 3, oder 4 Kohlenstoffatomen bedeuten, wobei R₃ in den obigen Formeln immer eine 2,2,6,6,-Tetramethylpiperidyl-4-gruppe ist und an Stelle der daran gebundenen -NH-Brücke auch eine Sauerstoff-Brücke oder ein Brückenglied der Formel -N(C₁₋₂-Alkyl)- stehen kann.
   Bevorzugte Komponenten B, freie Säuren zur Salzbildung mit basischen Aufhellern sind z.B.
35. H₂N-C₂₋₁₂-Alkylen-COOH,
36. 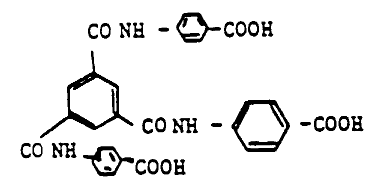
37. 4-Benzoylamino-benzoesäure,
38. HOOC-Alkylen 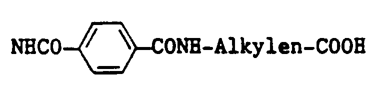
39. 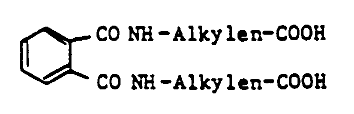
40. 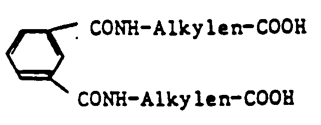
41. 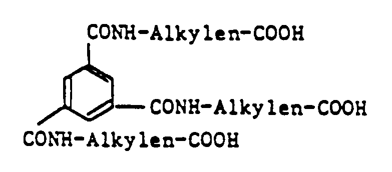
42. 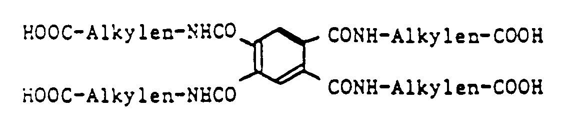
43. 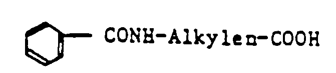
44. 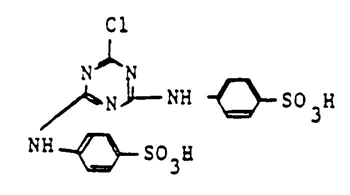
45. 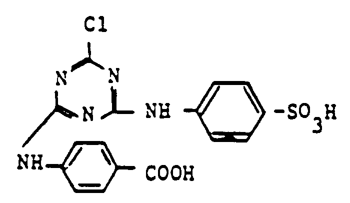
46. 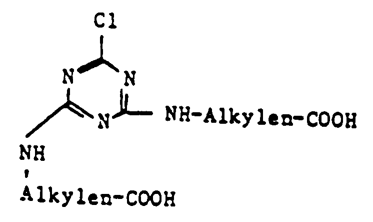
47. 2,4,-Bis-(4'-carboxyphenylamino)-6-chlortriazin,
48. 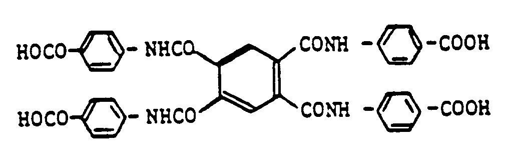
49. 1,4-Bis-(4'-carboxyphenylaminocarbonyl)-benzol,
50. 1,3-BiS-(4'-carboxyphenylaminocarbonyl)-benzol,
51. 1,2-Bis-(4'-carboxyphenylaminocarbonyl)-benzol,
52. Carboxy-C₁₋₃-alkylen-phenyl und
53. HOCO-R₇-COOH, worin R₇ die direkte Bindung oder ein C₁₋₁₂-Alkylenrest ist, wobei die genannten Alkyl-und Alkylenreste geradkettig oder verzweigt sein können und vorzugsweise (falls nichts anderes 1, 2, 3 oder 4, insbesondere 1 oder 2 Kohlenstoffatome enthalten.

Als Aufheller, die mit den obengenannten, bzw. mit den im Patentanspruch 1 definierten, als freie Basen oder Säuren vorliegenden Verbindungen unter Salzbildung die erfindungsgemässen Aufheller ergeben, kommen z.B. die Verbindungen der Formeln a₁ bis a₂₃ (saure Gruppen enthaltend) und b₁ bis b₅ in Betracht: 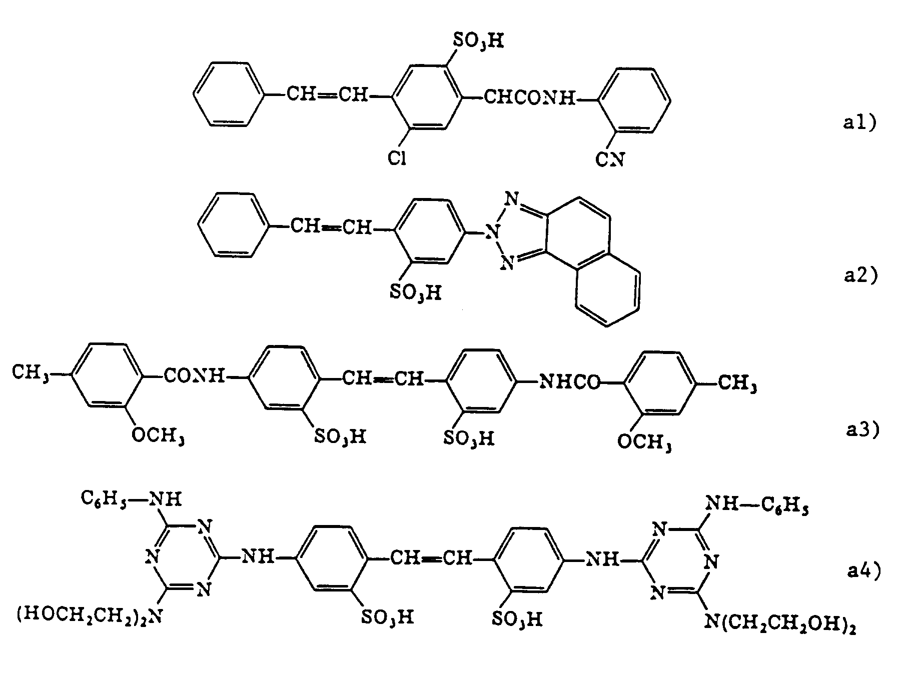 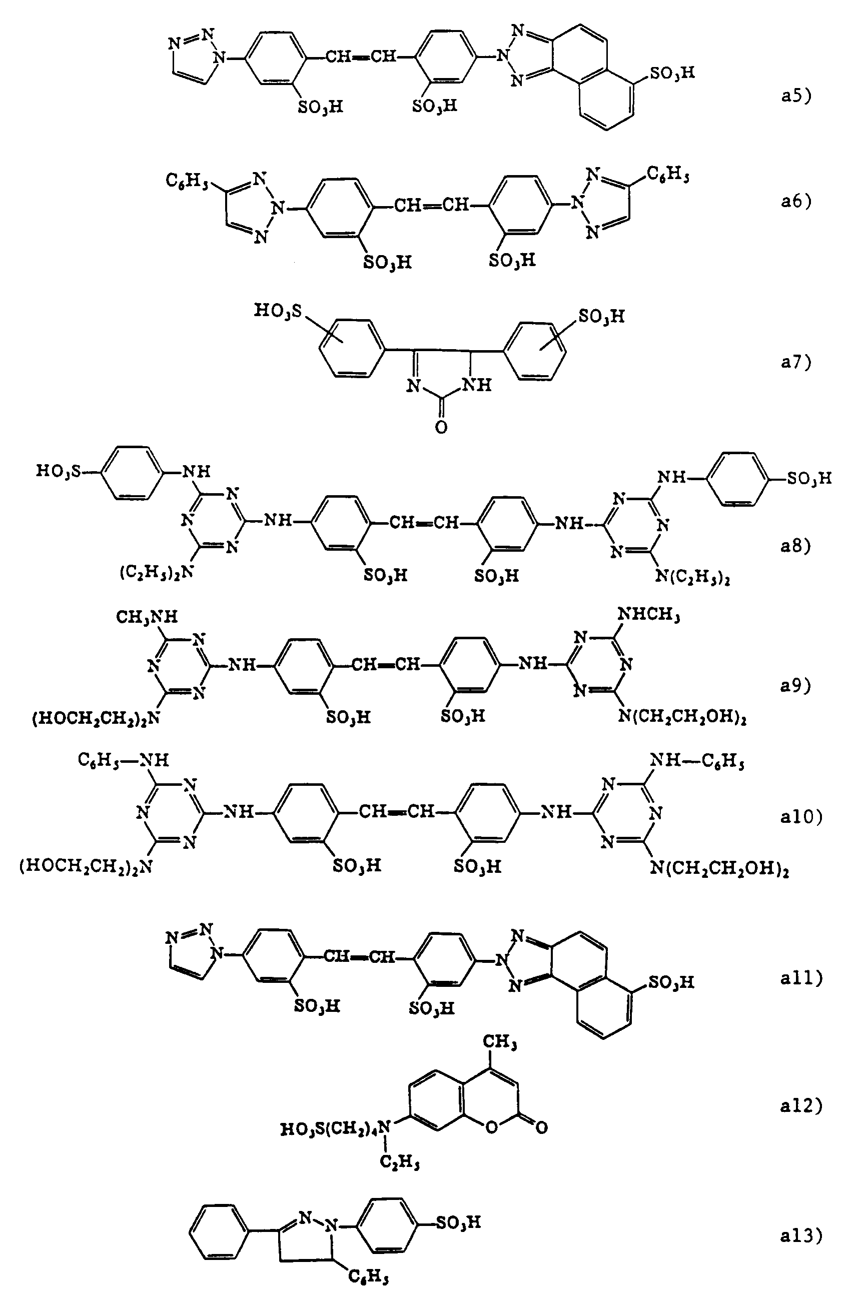 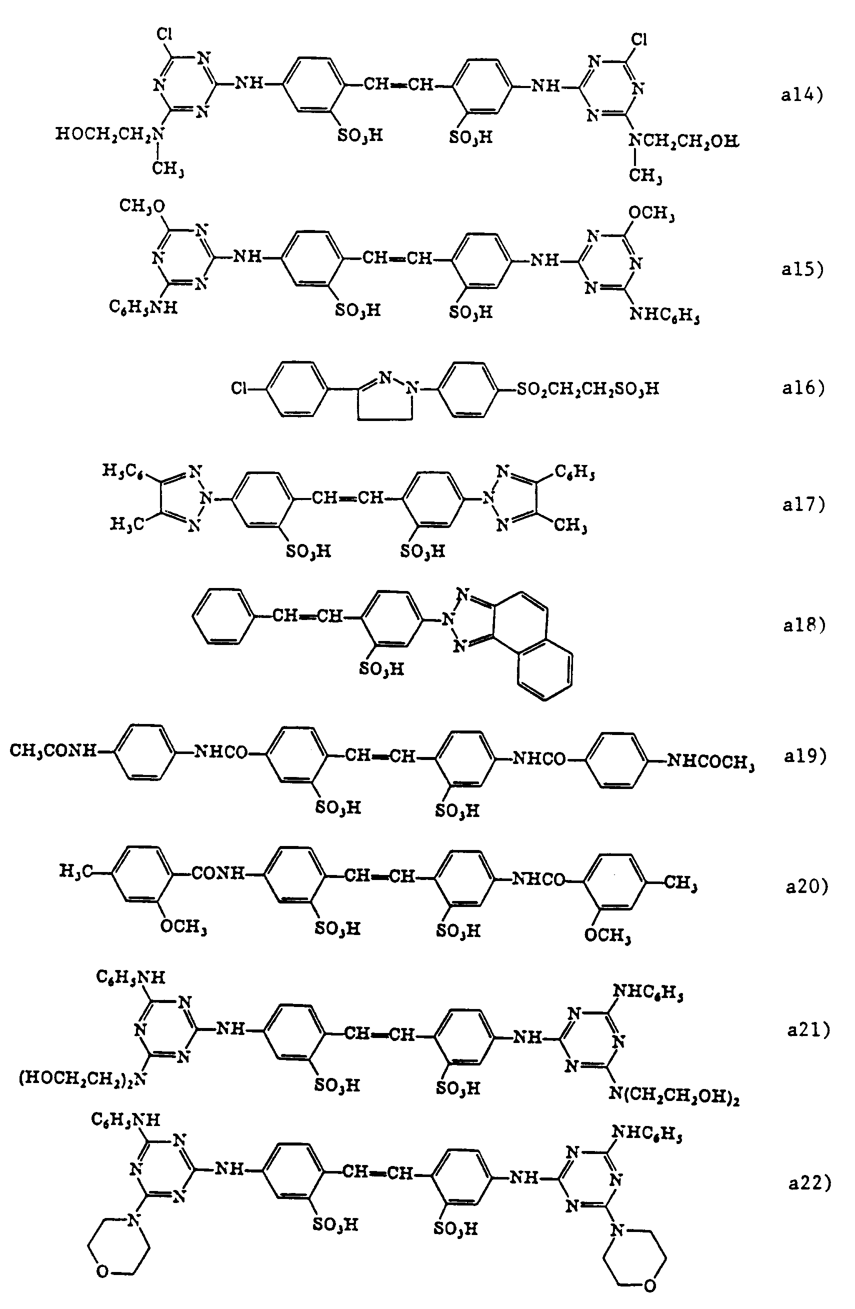 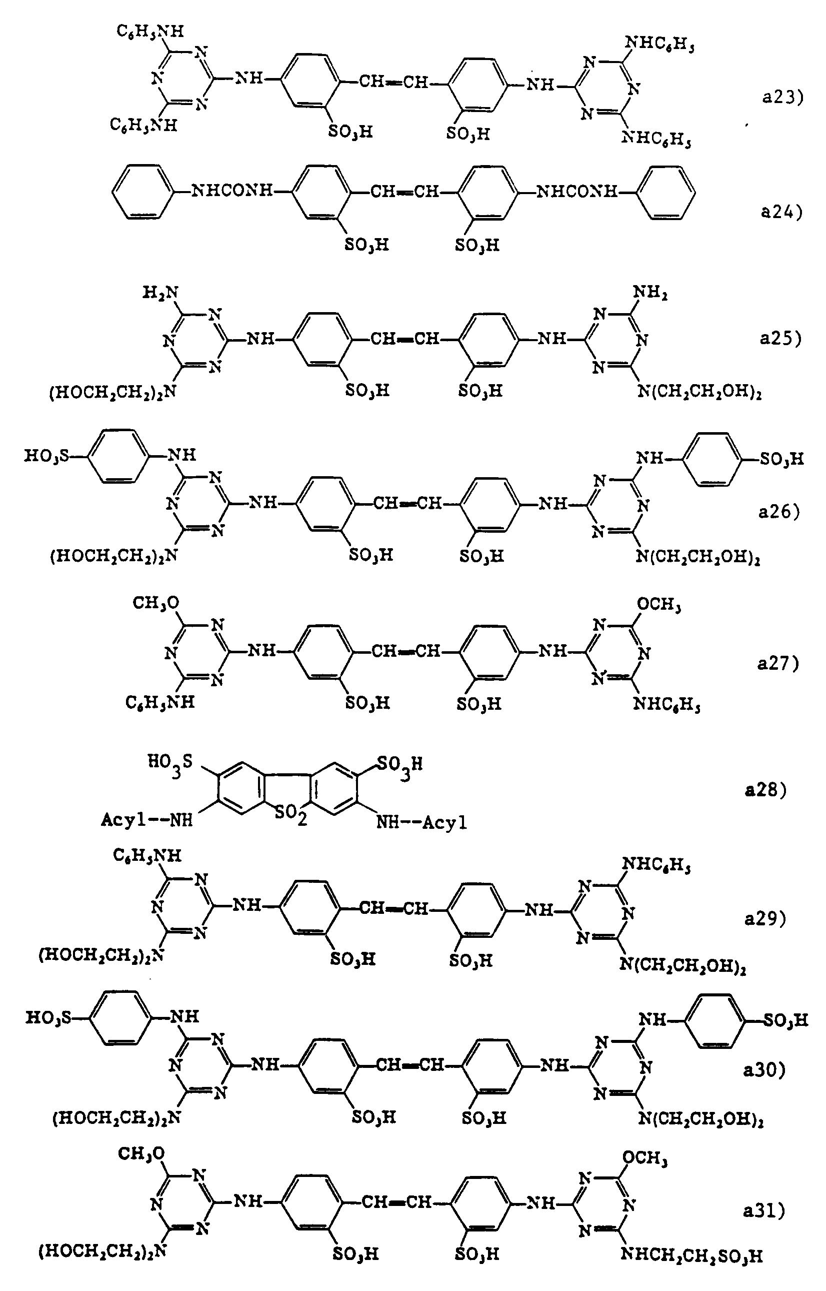 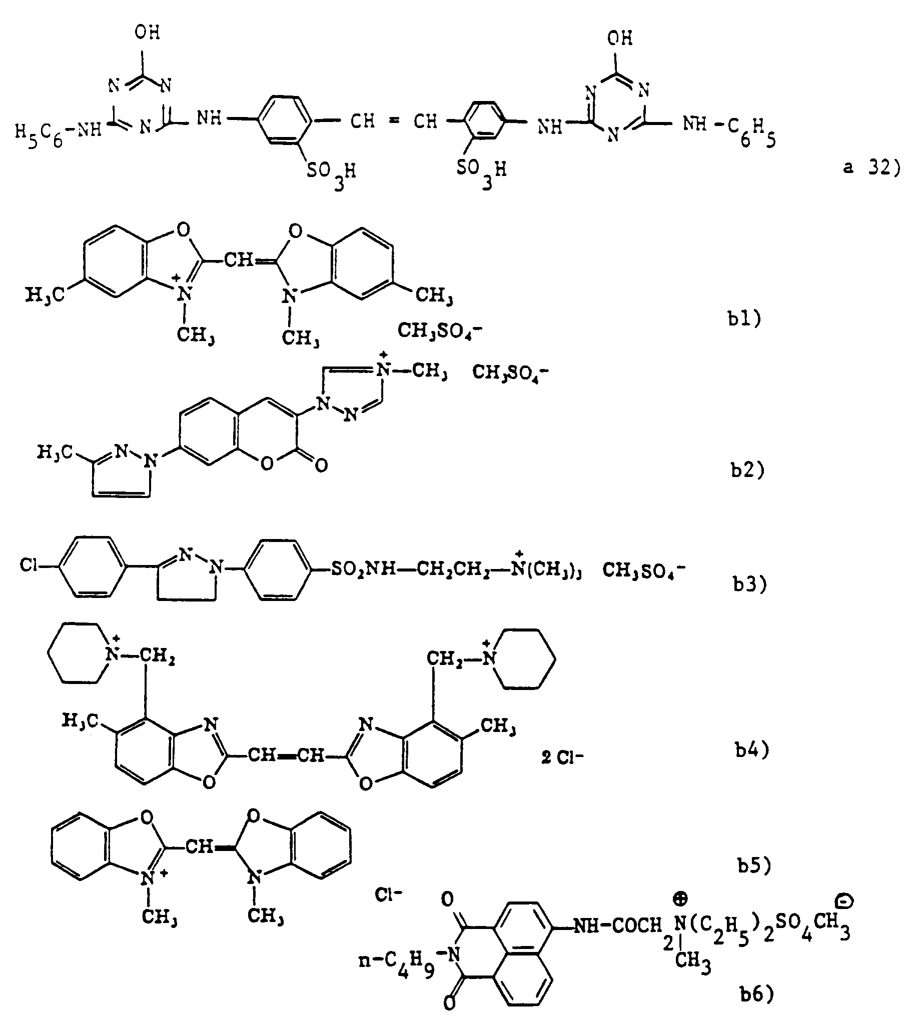
Die Herstellung der Komponenten B, der Amino-, bzw. Imino-Verbindungen, erfolgt gemäss dem Fachmann durchaus geläufigen Verfahren [Amidbildung aus dem Säurechlorid (z.B. Trimesinsäuretrichlorid, Bsp. 1) und einem Amin (z.B. 2,2,6,6-Tetramethyl-4-amino-piperidin), Kondensation von Aminen mit Halogentriazinyl etc.].

Die Herstellung der Verbindungen (Salze) der Formel I erfolgt ebenfalls auf eine beliebige, dem Fachmann geläufige Weise, die anionischen Verbindungen können im allgemeinen, so wie sie bei der Produktion anfallen (z.B. als Natriumsalze), in Gegenwart adäquater Mengen einer Mineralsäure (z.B. HCl) mit den basischen Komponenten B unter Salzbildung umgesetzt werden. Verden die freien Säuren eingesetzt, ist es nicht nötig Säure zuzusetzen. Die kationischen Verbindungen werden vorzugsweise in leicht alkalischem Milieu mit den saure Gruppen enthaltenden Komponenten B umgesetzt. Das Vermischen in der Masse und der Einsatz in gelösten Kunststoffen erfolgen analog zu bekannten Methoden auf dem Gebiet der Massefärbung, bzw. dem Gebiet der Lösungsmittelfarbstoffe.

Unter synthetischen Polyamiden sind sämtliche bekannten Kunststoffe dieser Art zu verstehen, insbesondere die Polykondensate oder Polymerisate aus Dicarbonsäuren und Diaminen, z.B. aus Adipinsäure und Hexamethylendiamin, aus Lactamen, z.B. ε-Caprolactam, oder aus Aminocarbonsäuren, z.B. ω-Aminoundecansäure. Auch die übrigen,z.B. im Buch "Synthesefasern", Verlag Chemie, Weinheim; Deerfield Beach Florida; Basel, 1981, (herausgegeben von Béla von Falkai), Abschnitt 6 "Charakterisierung synthetischer Fasern", beschriebenen, im allgemeinen schwer färbbaren synth. Polyamide lassen sich mit den Verbindungen der Formel I gut veredeln. Die mit den Verbindungen der Formel I vermischte Polyamidschmelze wird in üblicher Weise verformt, z.B. in Schmelzspinn-, Spritzguss-, Strangspritz- oder Folienblasmaschinen.

Falls nicht anders präzisiert, bedeuten in den folgenden Beispielen die Teile Gewichtsteile und die Prozente Gewichtsprozente. Die Temperaturen sind in Celsiusgraden angegeben.

### BEISPIEL 1

a) Auf bekannte Weise werden 184,5g 2,4,6-Trichlortriazin mit 314 g 2,2,6,6-Tetramethyl-4-aminopiperidin kondensiert.
b) 45,5 g einer Handelsform (85 % Aktivsubstanz) des Aufhellers der Formel 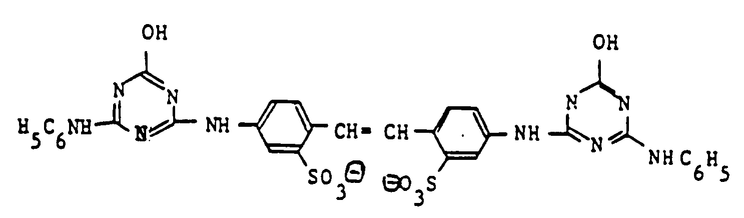 werden in 100 ml Wasser verrührt, mit NaOH (verdünnt) auf pH 12 gestellt und mit einem Gemisch aus 21,2 g des gemäss a) erhaltenen Kondensats [2,4-Bis-(2',2',6',6'-tetramethylpiperidyl-4'-amino)-6-chlortriazin], 50 ml Wasser und 10 ml HCl (30 %) langsam, unter Rühren, versetzt. Man rührt noch 1 Stunde, filtriert den erhaltenen Feststoff (das Piperidiniumsalz des Aufhellers) ab, wäscht ihn mit Wasser salzfrei und trocknet ihn.

### BEISPIEL 2

Auf analoge Weise wie im Beispiel 1 beschrieben, wird 1 Moläquivalent des Aufhellers der Formel b 2 (oben) mit 1 Moläquivalent der Verbindung der Formel 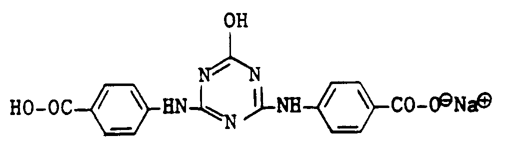
unter Salzbildung zur Reaktion gebracht, das unlösliche Salz abfiltriert, salzfrei gewaschen und getrocknet.

### BEISPIEL 3

122 Teile des Lichtstabilisators der Formel 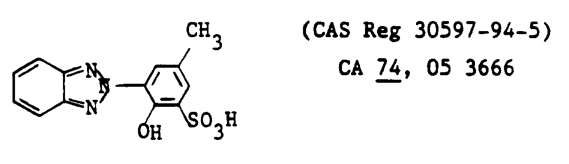
werden mit 88,4 Teilen Bis-(2,2,6,6-tetramethylpiperidyl-4)-terephthalsäurediamid (Verbindung 5, oben) in 500 Teilen Wasser verteilt und unter Rühren 5 Stunden auf 50° erwärmt. Das unlösliche Reaktionsprodukt wird abfiltriert, mit kaltem Wasser ausgewaschen und im Vakuum getrocknet. Man erhält so ein 2:1-Salz mit hervorragenden stabilisierenden Eigenschaften für synthetische Kunststoffe, insbesondere Polyamid.

### BEISPIEL 4

Man verfährt wie im Beispiel 3 angegeben, setzt aber nur 61 Teile Lichtstabilisator (wie oben) mit 84,7 Teilen der Verbindung 2 [=2,4-Bis-(2ʼ,2ʼ,6',6-tetramethylpiperidyl-4-amino)-6-chlortriazin] zu einem 1:1 Salz um und erhält so eine Verbindung mit ähnlichen Eigenschaften.

### BEISPIEL 5

Man verfährt wie im Beispiel 3 angegeben, setzt aber an Stelle der 88,4 Teile des Terephthalsäurediamids 161,2 Teile der Verbindung 21 (oben, Pyromellitsäuretetramid) ein. Das so erhaltene Salz hat wie diejenigen der Beispiele 3 und 4 ausgezeichnete lichtstabilisierende Eigenschaften und migriert auch unter extremen Bedingungen nicht aus dem mit ihm stabilisierten Kunststoff heraus.

### BEISPIEL 6

Man verfährt wie im Beispiel 5 angegeben, verwendet aber nur 61 Teile des Lichtstabilisators (Benztriazolverbindung). Das erhaltene Produkt ist in seinen Eigenschaften dem der Beispiele 3 bis 5 sehr ähnlich.

### BEISPIEL 7

Analog zur Arbeitsweise des Beispiels 3 setzt man 67,3 Teile der Verbindung der Formel 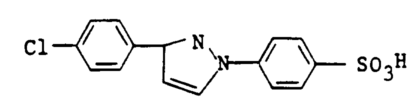
und 84,7 Teile 2,4-bis-(2',2',6',6',-tetramethylpiperidyl-4-amino)-6-chlortriazin (=Verbindung 2, oben) zu einem Salz um. Man erhält so einen sehr waschechten Aufheller, der auch die Lichtstabilität der mit ihm versetzten Polymere sehr positiv beeinflusst.

### BEISPIEL 8

Man verfährt wie im Beispiel 7 angegeben, setzt jedoch 134,6 Teile der Pyrazolinverbindung ein. Es resultiert ein sehr wirksamer Aufheller, der auch die Lichtstabilität der mit ihm versetzten Polymere sehr verbessert.

### BEISPIEL 9

Man verfährt wie im Beispiel 7 angegeben, setzt aber an Stelle der 84,7 Teile der Triazinverbindung 88,4 Teile der Verbindung der Formel 5 (Beispiel 3) ein und erhält so einen Aufheller mit Lichtechtheit-verbessernden Eigenschaften.

### BEISPIEL 10

Nach der Vorschrift des Beispiels 9, jedoch mit 134,6 Teilen (an Stelle von 67,3 Teilen) der Pyrazolinverbindung erhält man einen Aufheller, der, in Polyamidmassen eingebracht, sehr licht- und waschecht ist.

### BEISPIEL 11

Gemäss den Angaben im Beispiel 7, jedoch mit 161,2 Teilen der Verbindung 21 (Pyromellitsäuretetramid) erhält man ebenfalls einen Aufheller mit verbesserter Licht- und Waschechtheit.

### BEISPIEL 12

Nach den Angaben im Beispiel 8, aber mit 161,2 Teilen der Verbindung 21 (Pyromellitsäuretetramid) an Stelle der 84,7 Teile Triazinverbindung, erhält man einen Aufheller mit sehr ähnlichen Eigenschaften.

Analog zu den Angaben in den vorhergehenden Beispielen können auch die Verbindungen der Formeln 1 bis 34 mit saure Gruppen enthaltenden Aufhellern (Formeln a₁ bis a₃₁), bzw. die Verbindugnen der Formeln 35 bis 55 mit basischen Aufhellern (der Formeln b₁ bis b) zu den erfindungsgemässen Aufhellersalzen umgesetzt werden.

### APPLIKATIONSBEISPIEL 1

100 Teile Poly-ε-caprolactam in Form eines Pulvers werden mit 1 Teil des Aufhellersalzes gemäss Beispiel 1 in einem Trommelmischer vermischt. Das Pulver verteilt sich nach kurzer Zeit sehr gleichmässig. Nach ca. 10 Minuten wird das Gemisch 16 Stunden bei 120°C getrocknet, in eine Schmelzspinnmaschine eingebracht und nach 8-minütiger Verweilzeit bei 275-280°C unter Stickstoffatmosphäre zu Fasern versponnen. Der erhaltene Aufhelleffekt ist ausserordentlich licht- und waschecht. Bei einem vergleichenden Waschechtheits-Test hat sich gezeigt, dass der Aufhellungseffekt der erfindungsgemäss behandelten Fasern nach dem Waschen (1% Waschmittel, 1 Stunde bei 70°) mehr als 12 mal so stark war wie der von Fasern, die mit dem nicht (erfindungsgemäss) versalzten Aufheller in der Masse vermischt wurden.

### APPLIKATIONSBEISPIEL 2

In 95 Teile eines Nitrocellulose-Lacks, hergestellt aus 18,8 % mit 35 % Isopropanol angefeuchtete Nitrocellulose A15 (in Form weisser Flocken),
6,3 % Acrylsäurebutylester-Polymere, Weichmacherharz (Acronal 4F, BASF),
3,3 % Diphenyloctylphosphat, Weichmacher (Santicizer 141, Monsanto),
10,0 % Methoxypropanol (Dowanol PM, Dow Chemical),
41,6 % Aethanol
10,0 % Aethoxypropanol und
10,0 % Aethylacetat,
wurden 5 Teile des Farbstoffsalzes gemäss Beispiel 2 mit dem Dissolver eingerührt und über Nacht auf dem Rollbock gelöst. Die Löslichkeit ist perfekt.

Auf a) unlackierte und b) mit farblosem Nitrocelluloselack lackierte Aluminiumfolie wurden mit dem 25 µm-Ziehstab Nassfilme mit der obigen Lackkomposition hergestellt, diese 5 Stunden bei 130°C getrocknet und die Transparenz als Maßstab der Verträglichkeit des Farbstoffsalzes mit dem lösungsmittelfreien Film, und die Haftfestigkeit nach dem Scotch-tape-Test auf der unlackierten und der vorher lackierten Aluminumfolie bestimmt. Beide Eigenschaften wurden als perfekt beurteilt.

## Patentansprüche

1. Optische Aufheller und/oder Lichtstabilisatoren der allgemeinen Formel I
Aₙ . Bₙ (I)
worin
A der Rest eines a) anionischen oder kationischen Aufhellers oder Lichtstabilisators,
B im Fall einen mindestens eine Ammonium-, Cycloammonium- oder Immoniumgruppe und mindestens eine aliphatische, cycloaliphatische, an mindestens eine Carbonylgruppe gebundene aromatische oder eine heteroaromatische Gruppe aus der Reihe der Triazin-, Pyrimidin-, Chinazolin-, Chinoxalin-, Piperazin-, Phthalimid- oder Phthalazinverbindungen enthaltenden Rest, wobei die eine aliphatische oder cycloaliphatische Gruppe enthaltenden Reste auch mindestens eine cyclisch gebundene Ammoniumgruppe enthalten
und im Fall b) einen mindestens einen Carbonsäure- oder Sulfonsäurerest und eine aliphatische, cycloaliphatische, aromatische oder hetero-aromatische Gruppe aufweisenden Rest oder einen C₂₋₁₄-Di-carbonsäure- oder Amino-C₂₋₁₂-alkyl-carbonsäurerest,
und die beiden n unabhängig voneinander 1, 2, 3 oder 4 bedeuten, wobei A und B salzartig aneinander gebunden sind.

2. Die Verbindungen der Formel I gemäss Anspruch 1, worin
A der Rest eines anionischen Aufhellers oder Lichtstabilisators,
B eine oder zwei Ammonium- oder Immoniumgruppen und ein C₁₋₁₂-Alkyl-, Cyclohexan-, Piperazin-, Triazin-, Pyrimidin-, Chinazolin-, Chinoxalin-, Phthalazin-, Phthalimid- oder Benzol-Brückenglied enthaltender Rest, vorzugsweise ein solcher der Benzol- oder Triazinreihe und die beiden
ein n=1 und das andere n 1 oder 2, vorzugsweise 1 bedeuten.

3. Die Aufheller und Lichtstabilisatoen der Formel I, gemäss Anspruch 1 oder 2,
worin
A ein Rest der Formel 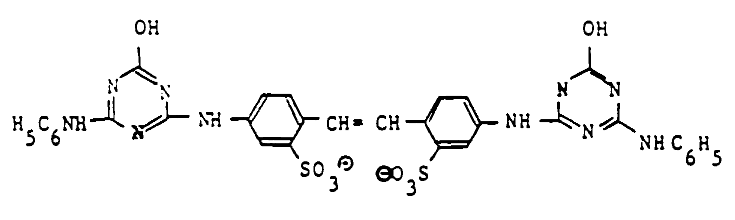 ist.

4. Die Aufheller und Lichtstabilisatoren der Formel I, gemäss einem der vorhergehenden Ansprüche, worin A der Rest eines anionischen Aufhellers und B ein Rest ist, der eine oder zwei 2,2,6,6-Tetramethylpiperidinium- und gegebenenfalls eine, zwei oder drei 2,2,6,6-Tetramethylpiperidylgruppen, immer in 4-Stellung gebunden, trägt.

5. Die Aufheller und Lichtstabilisatoren der Formel I, gemäss einem der vorhergehenden Ansprüche, worin
A den Rest eines anionischen Aufhellers oder Lichtstabilisators
B mit einer, zwei oder drei 2,2,6,6-Tetramethylpiperidyl-4-oxycarbonyl- oder -amino-carbonylgruppen (f) und einer über eine -CO-Brücke gebundenen Gruppe der Formel g 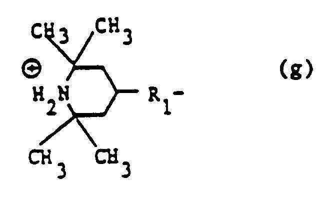 R₁ = -O- oder -NR₂ und R₂ = H oder C₁₋₂-Alkyl, substituiertes Benzol, oder Triazin, das in 2-Stellung eine Gruppe der Formel g, in 4-Stellung eine Gruppe der Formel f und in 6-Stellung ein Chlor oder ebenfalls eine Gruppe der Formel f als Substituenten trägt oder eine Verbindung der Formel 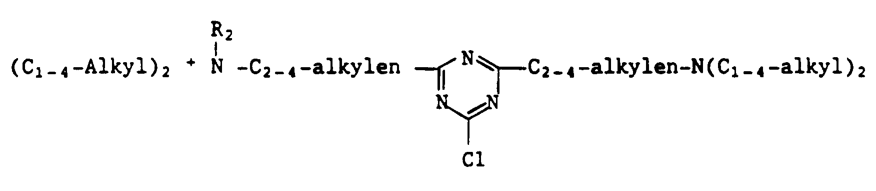 und n 1 bedeuten.

6. Die Verwendung der Verbindungen der Formel I gemäss einem der vorhergehenden Ansprüche zum Aufhellen und Lichtstabilisieren von synthetischen Polyamiden oder Lösungsmittel enthaltenden Lacksystemen.

## Claims

1. The optical brighteners and/or light stabilizers of the general formula I
Aₙ . Bₙ (I)
wherein
A is the radical of an a) anionic or b) cationic brightener or light stabilizer,
B in the case of a) is a radical containing at least one ammonium, cycloammonium or immonium group and at least one aliphatic group, cycloaliphatic group, or aromatic group bonded to at least one carbonyl group, or heteroaromatic group from the series of triazine, pyrimidine, quinazoline, quinoxaline, piperazine, phthalimide or phthalazine compounds, whereby the radicals containing an aliphatic or cycloaliphatic group also contain at least one cyclically bonded ammonium group,
and in the case of b) is a radical having at least one carboxylic acid or sulphonic acid radical and one aliphatic, cycloaliphatic, aromatic or heteroaromatic group, or is a C₂₋₁₄-dicarboxylic acid or amino-C₂₋₁₂-group, or is a C₂₋₁₄-dicarboxylic acid or amino-C₂₋₁₂-alkylcarboxylic acid radical,
and both n, independently of one another,are 1, 2, 3 or 4, whereby A and B are bonded together like a salt.

2. The compounds of formula I according to claim 1, wherein
A is the radical of an anionic brightener or light stabilizer,
B is a radical containing one or two ammonium groups and one C₁₋₁₂-alkyl, cyclohexane, piperazine, triazine, pyrimidine, quinazoline, quinoxaline, phthalazine, phthalimide or benzene bridging member, preferably one from the benzene or triazine series, and both
one n=1 and the other n is 1 or 2, preferably 1.

3. The brighteners and light stabilizers of formula I, according to claim 1 or 2,
wherein A is a radical of formula

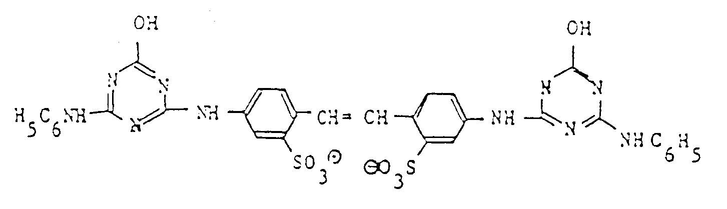

4. The brighteners and light stabilizers of formula I, according to one of the preceding claims, wherein A is the radical of an anionic brightener and B is a radical which bears one or two 2,2,6,6-tetramethylpiperidinium groups and optionally one, two or three 2,2,6,6-tetramethylpiperidyl groups, always in 4-position.

5. The brighteners and light stabilizers of formula I, according to one of the preceding claims,
wherein
A is the radical of an anionic brightener or light stabilizer
B is benzene which is substituted by one, two or three 2,2,6,6-tetramethylpiperidyl-4-oxycarbonyl or -amino-carbonyl groups (f), and by a group, bonded by a -CO- bridge, of formula g 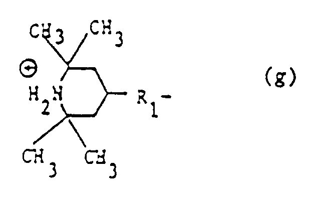 R₁ = -O- or -NR₂- and R₂ = H or C₁₋₂-alkyl
or it is triazine which bears as substituents in 2-position a group of formula g, in 4-position a group of formula f and in 6-position a chlorine or similarly a group of formula f, or it is a compound of formula 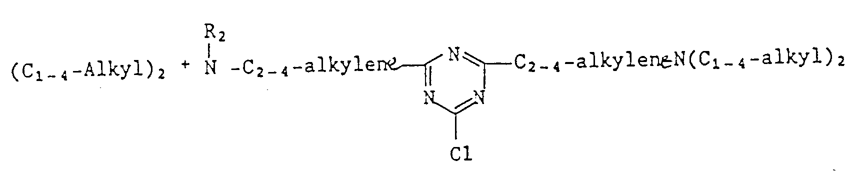 and n is 1.

6. The use of the compounds of formula I according to one of the preceding claims for the brightening and light stabilizing of synthetic polyamides or solvent-containing lacquer systems.

## Revendications

1. Azurants optiques et/ou photostabilisants de formule générale I
Aₙ . Bₙ (I)
dans laquelle
A signifie le reste d'un azurant optique ou d'un photostabilisant a) anionique ou b) cationique,
B signifie,
dans le cas a), un reste contenant au moins un groupe ammonium, cycloammonium ou iminium et au moins un groupe aliphatique, un groupe cycloaliphatique, un groupe aromatique lié à au moins un groupe carbonyle ou un groupe hétéroaromatique choisi parmi la triazine, la pyrimidine, la quinazoline, la quinoxaline, la pipérazine, le phtalimide et la phtalazine, les restes qui contiennent un groupe aliphatique ou cycloaliphatique contenant aussi au moins un groupe ammonium lié en formant un cycle, et dans le cas b), un reste comportant au moins un groupe carboxylique ou sulfo et un groupe aliphatique, cycloaliphatique, aromatique ou hétéro-aromatique, ou un reste d'un acide di-carboxylique en C₂-C₁₄ ou d'un acide amino-alkyl en C₂-C₁₂-carboxylique, et
les deux symboles n signifient, indépendamment l'un de l'autre, 1,2,3 ou 4,
A et B étant liés l'un à l'autre de manière à former un sel.

2. Les composés de formule I selon la revendication 1, dans lesquels
A signifie le reste d'un azurant optique anionique ou d'un photostabilisant anionique,
B signifie un reste contenant un ou deux groupes ammonium ou iminium et un pont alkylique en C₁-C₁₂, cyclohexanique, pipérazinique, triazinique, pyrimidinique, quinazolinique, quinoxalinique, phtalazinique, phtalimidique ou benzénique, de préférence un tel reste de la série du benzène ou de la triazine,
et parmi les deux n l'un est égal à 1 et l'autre signifie 1 ou 2, de préférence 1.

3. Les azurants optiques et les phtostabilisants de formule I selon la revendication 1 ou 2,
dans lesquels A signifie un reste de formule

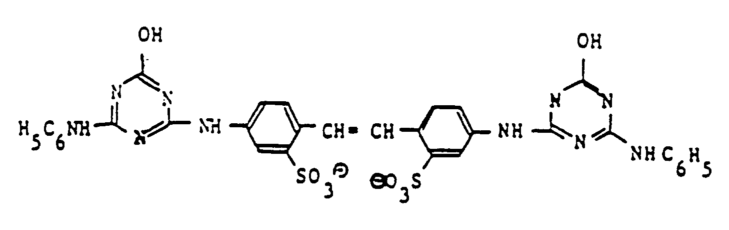

4. Les azurants optiques et les photostabilisants de formule I, selon l'une des revendications précédentes, dans lesquels A signifie le reste d'un azurant optique anionique et B signifie un reste qui porte un ou deux groupes 2,2,6,6-tétraméthyl-pipéridinium et éventuellement un, deux ou trois groupes 2,2,6,6-tétraméthylpipéridyle, liés toujours en position 4.

5. Les azurants optiques et les photostabilisants de formule I, selon l'une des revendications précédentes, dans lesquels
A signifie le reste d'un azurant optique anionique ou d'un photostabilisant anionique,
B signifie un benzène substitué par un, deux ou trois groupes (f) qui sont des groupes 2,2,6,6-tétraméthylpipéridyl-4-oxycarbonyle ou -amino-carbonyle, et par un groupe de formule g 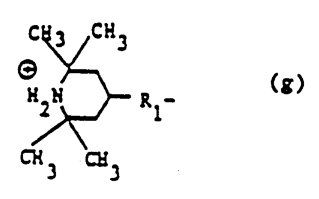 où R₁ = -O- ou -NR₂ et R₂ = H ou alkyle en C₁-C₂, lequel groupe de formule g est lié par l'intermédiaire d'un pont -CO-, ou signifie une triazine qui porte, comme substituants, en position 2 un groupe de formule g, en position 4 un groupe de formule f et en position 6 un atome de chlore ou également un groupe de formule f, ou bien B signifie un composé de formule 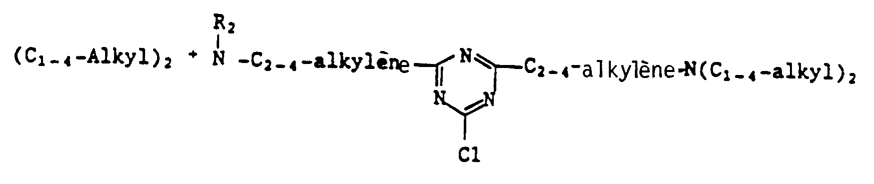 et n signifie 1.

6. L'utilisation des composés de formule I selon l'une des revendications précédentes, pour l' azurage optique et la photostabilisation des polyamides synthétiques ou des vernis contenant des solvants.
